# EUROPEAN PATENT APPLICATION

(11) **EP 4 122 961 A1**
(43) Date of publication of application: **25.01.2023**
(21) Application number: 21186790.8
(22) Date of filing: 20.07.2021
(51) Int. Cl.: C08B 37/08, A61K 31/722

(54) **CHITOSAN OLIGOMERS AND USES THEREOF**

(71) Applicant: Westfälische Wilhelms-Universität Münster, 48149 Münster (DE)
(72) Inventor: MOERSCHBACHER, Bruno, 48161 Münster (DE); CORD-LANDWEHR, Stefan, 48159 Münster (DE); REGEL, Eva Katharina, 48149 Münster (DE); RICHTER, Carolin, 48329 Havixbeck (DE)
(74) Representative: Viering, Jentschura & Partner mbB Patent- und Rechtsanwälte

(57) **Abstract**

The present invention relates to chitosan oligomers obtainable by enzymatic hydrolysis of chitosan polymers, compositions containing them, and uses thereof.

## Description

### Filed of the invention

The present invention relates to chitosan oligomers, compositions containing them, and uses thereof.

### Background

Chitosans are a family of functional biopolymers, derived from the partial de-*N*-acetylation of chitin, one of the most abundant biopolymers in the world. Whereas chitin, a linear homopolymer of β-1,4-glycosidically linked *N*-acetyl-D-glucosamine (GlcNAc) residues, forms crystalline fibers and is insoluble in aqueous solvents, chitosans are soluble at slightly acidic pH values, due to the positive charges conveyed by their de-*N*-acetylated D-glucosamine (GlcN) residues. The unique polycationic nature of the GlcN residues combined with the hydrophobic patches formed by the GlcNAc residues within the chitosan chains is at least partially responsible for the many bioactivities of chitosans, such as their antimicrobial, plant-strengthening, and wound-healing activities, which make them extremely valuable compounds for applications in biomedicine, agriculture, cosmetics and in the food industry. The structure of chitosans can be described by three parameters: the degree of polymerization (DP), the degree of acetylation (DA), and the pattern of acetylation (P_{A}), the latter referring to the sequence of GlcN and GlcNAc units. These three characteristics influence strongly their physico-chemical solution properties as well as their biological functionalities.

To date chitosan polymers are either prepared by means of homogeneous or heterogeneous partial alkaline de-*N*-acetylation of chitin or by means of homogeneous partial re-*N*-acetylation of polyglucosamine. Partially acetylated chitosans generated via chemical *N*-acetylation or de-N-acetylation of GlcN or GlcNAc subunits within the polymers, respectively, typically display random patterns of acetylation. However, the acetylation pattern is one of the properties of chitosans that is believed to crucially influence various biological activities. Strategies to avoid these random acetylation patterns and generate non-random chitosan polymers are described in international patent publication WO 2019/242847 A1.

Chitosan polymers are interesting targets for a variety of applications. For example, chitosans with a low degree of acetylation, and, as a consequence, with a relatively high density of positive charges, are interesting as carriers, for example in form of nanoparticles for drug, gene or vaccine delivery. Furthermore, chitosans are known to act as plant protectants, plant strengtheners or plant biostimulants, with these effects however being dependent inter alia on their pattern of acetylation. Moreover, chitosans have antimicrobial activities, hindering the growth of bacteria and fungi without being bactericidal or fungicidal, and thus may be of use e.g. in medicinal, cosmetics and food applications.

However, although various chitosan polymers and applications thereof are known, there is still need for further improved chitosan-derived agents and compositions that provide for increased efficiency for certain applications, in particular agricultural and biomedical applications.

### Summary of the Invention

The present invention is based on the inventors' finding that the diverse properties of chitosan polymers can be attributed to certain structural features. By generating specific non-random fragments of chitosan polymers, namely chitosan oligomers with a comparable low degree of polymerization, the known different properties of the polymer can be essentially limited to one functionality of choice. It thus becomes possible to design compositions that predominantly have a single one of chitosans' many different activities, while all other activities are suppressed. This is particularly advantageous for the plant stimulating properties on the one hand and the antimicrobial properties on the other hand.

In a first aspect, the present invention is therefore directed to chitosan oligomers obtainable by enzymatic hydrolysis of a chitosan polymer by a chitosan hydrolase, wherein the chitosan oligomers have a degree of polymerization (DP) of 30 or less, preferably 20 or less.

In various embodiments, the to-be-hydrolyzed chitosan polymer has a degree of acetylation (DA) of between 10 and 30%, preferably 15 to 25%. The degree of polymerization (DP) may be 60 or more, 100 or more, or 150 or more.

In various embodiments, the chitosan polymer used is obtainable by deacetylation of chitin polymers, including enzymatic and alkaline deacetylation, with alkaline deacetylation being the usual and thus preferred option. Alternatively, the chitosan polymer may also be obtained by partial acetylation of poly-D-glucosamine. The preferred option is the (partial) deacetylation of chitin.

In various embodiments, the chitosan hydrolase is a chitosanase. The chitosanase may specifically recognize and cleave the motif
(1) [X]ₙDD I X[X]ₙ, or
(2) [X]ₙXD I D[X]ₙ, or
(3) [X]ₙDX I X[X]ₙ,
wherein X is Glucosamine (GlcN) or *N*-Acetyl-Glucosamine (GlcNAc), D is Glucosamine (GlcN), n is 0 or an integer of from 1 to 5000, preferably 1 to 1000, and I indicates the cleavage site/position. The chitosan oligomers thus obtained may have the structure:
(1) [X]ₙDD,
(2) D[X]ₙXD, preferably D[X]ₙAD, and/or
(3) [X]ₙDX,
wherein X is Glucosamine (GlcN) or *N*-Acetyl-Glucosamine (GlcNAc), D is Glucosamine (GlcN), A is N-Acetyl-Glucosamine (GlcNAc) and n is 0 or an integer of from 1 to 20, preferably 1 to 10. In various embodiments,
(1) the oligomers have the structure [X]ₙ₋₁ADD, wherein [X]ₙ₋₁ is free of DD motifs or is [A]ₙ₋₁;
(2) the oligomers have the structure D[X]ₙAD, wherein [X]ₙ is free of DD motifs or is [A]ₙ; and/or
(3) the oligomers have the structure [X]ₙDX, wherein [X]ₙ is [A]ₙ.

In various embodiments, the chitosan oligomers have a degree of polymerization (DP) of 16 or less, more preferably 2-15.

In various embodiments, the chitosan oligomers generated by action of a chitosanase have a degree of acetylation (DA) less than that of the chitosan polymer they are obtained from, preferably of less than 20%, more preferably of less than 15%, most preferably of 10% or less. In various embodiments, the chitosan oligomers comprise at least one *N*-Acetyl-Glucosamine (GlcNAc) unit.

In various embodiments, the chitosan hydrolase may be a chitinase. The chitinase may specifically recognize and cleave the motif
(1) [X]ₙXA I X[X]ₙ,
(2) [X]ₙAX I A[X]ₙ, or
(3) [X]ₙAA I A[X]ₙ,
wherein X is Glucosamine (GlcN) or *N*-Acetyl-Glucosamine (GlcNAc), A is *N*-Acetyl-Glucosamine, n is 0 or an integer of from 1 to 5000, preferably 1 to 1000, and I indicates the cleavage site/position. The thus obtained chitosan oligomers may have the structure:
(1) [X]ₙXA,
(2) A[X]ₙAX, and/or
(3) A[X]ₙAA,
wherein X is Glucosamine (GlcN) or *N*-Acetyl-Glucosamine (GlcNAc), D is Glucosamine (GlcN), A is *N*-Acetyl-Glucosamine (GlcNAc) and n is 0 or an integer of from 1 to 20, preferably 1 to 10. In various embodiments,
(1) the oligomers have the structure [X]ₙDA, wherein [X]ₙ is [D]ₙ;
(2) the oligomers have the structure A[X]ₙAD, wherein [X]ₙ is [D]ₙ; and/or
(3) the oligomers have the structure A[X]ₙ₋₁DAA, wherein A[X]ₙ₋₁ is free of AAA motifs.

These chitosan oligomers may have a degree of polymerization (DP) of 16 or less, more preferably 2-15. In various embodiments, the oligomers generated by action of a chitinase have a degree of acetylation (DA) greater than that of the chitosan polymer they are obtained from, preferably of more than 20%, more preferably more than 25%, most preferably of 30% or more.

In various embodiments, the chitosan oligomers of the invention comprise a plurality or multitude of different oligomers produced by hydrolysis of a chitosan polymer. In such embodiments, the chitosan oligomers may be a mixture of chitosan oligomers that differ in length and/or structure. In such embodiments, the DP range is preferably met by all or essentially all oligomers of the mixture.

In various embodiments, the mixture of chitosan oligomers comprises less than 50% by weight of a chitosan polymer with a DP equal to or greater than 50.

In various embodiments, the chitosan oligomers or mixtures thereof are obtained by a separation step after hydrolysis of the chitosan polymer to reduce the amount of residual polymer and/or separate the chitosan oligomers from the enzymes used. In various such embodiments, the chitosan oligomers are essentially free of polymeric chitosan with a DP greater than 50.

In another aspect, the present invention relates to a composition comprising the chitosan oligomers described herein. Such a composition may be obtainable by isolating the oligomeric fraction of a chitosan polymer hydrolysate.

In addition to the chitosan oligomers the composition may further comprise additional components, such as at least one auxiliary or excipient. Also included are diluents or solvents.

In still another aspect, the invention is directed to the use of a composition comprising the chitosan oligomers of the invention or the compositions described herein, wherein the chitosan oligomers are obtained by hydrolysis of a chitosan polymer with a chitosanase, as a plant biostimulant.

In a still further aspect, the invention relates to the use of a composition comprising the chitosan oligomers of the invention or the compositions described herein, wherein said chitosan oligomers are obtained by hydrolysis of a chitosan polymer with a chitinase, as an antimicrobial agent and/or biopesticide.

The composition comprising the chitosan oligomers described herein, in particular those comprising oligomers obtainable by hydrolysis of a chitosan polymer with a chitinase, may be used as a medicament or may be used in the treatment of microbial infection. Said infection may be in a subject, for example a mammal, such as a human.

### Brief description of the figures

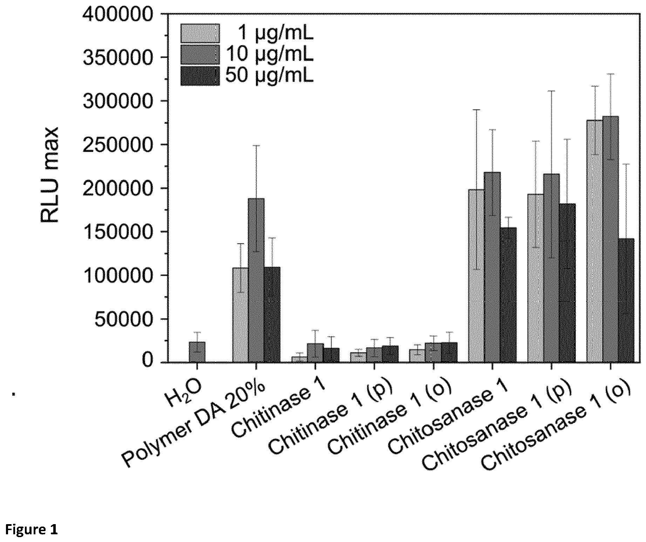

**Figure 1** shows induction of resistance in *Arabidopsis thaliana* seedlings by chitosan polymer (degree of acetylation about 20%) and its enzymatic hydrolysates (chitinase or chitosanase) and the purified polymeric (p) and oligomeric (o) fractions of the hydrolysate. Shown are mean values ± standard deviation (n = 4) of one representative experiment out of three independent experiments.

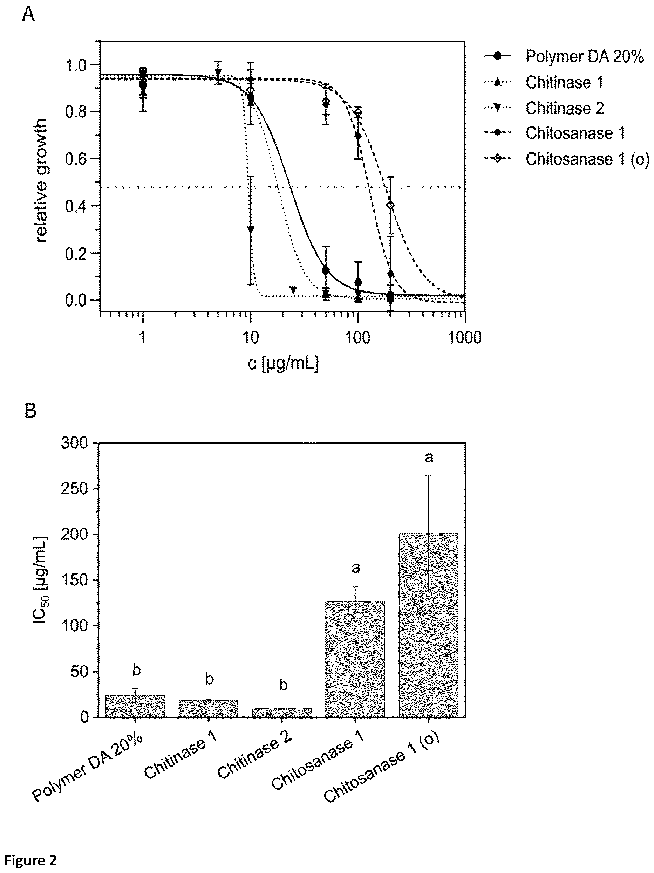

**Figure 2** shows the antimicrobial activity of a chitosan polymer (DA 20%), its enzymatically generated hydrolysates (chitinases 1 and 2; chitosanase 1) and the purified oligomeric fraction (chitosanase 1 (o)) against *Fusarium graminearum.* A: Dose-dependent effects between the chitosan concentration and the relative fungal growth. B: Half maximal inhibitory concentration (IC₅₀). Shown are the mean values (± standard deviation) of three independent experiments.

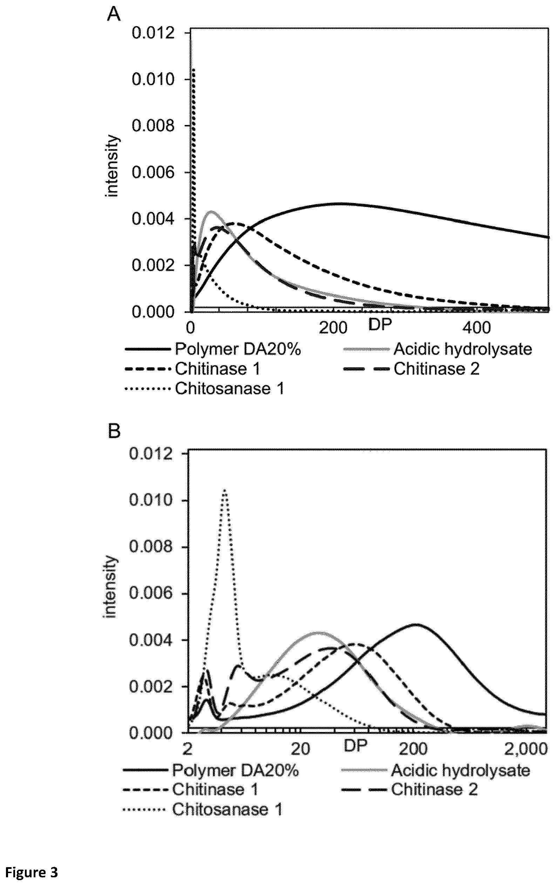

**Figure 3** shows the results of analytic size exclusion chromatography (HP-SEC) for determination of the number average degree of polymerization (DP) and the DP distribution of the starting chitosan polymer (DA 20%) and its acidic and enzymatic hydrolysates.

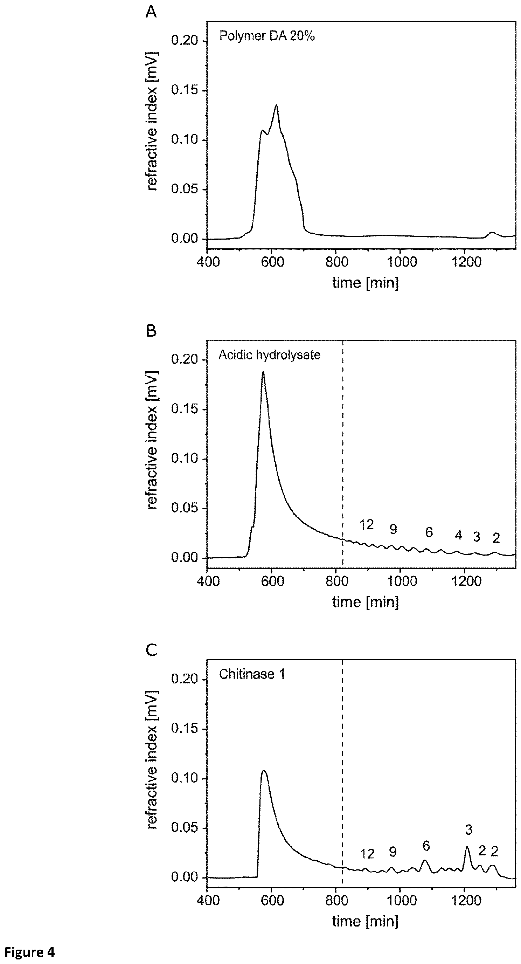

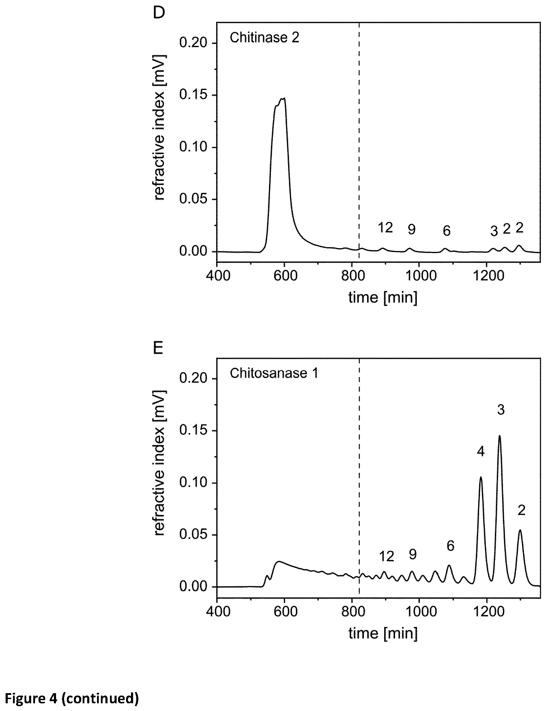

**Figure 4** shows the results of semi-preparative size exclusion chromatography (prepSEC) for analysis and separation of polymeric and oligomeric fractions (the dashed line indicates the threshold set for oligomers between DP 15 and 16) of the starting polymer (A) and its acidic (B) and enzymatic hydrolysates with chitinase (C, D) or chitosanase (E).

### Detailed Description

Unless otherwise defined, all terms of art, notations and other scientific terminologies used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. The techniques and procedures described or referenced herein are generally well understood and commonly employed using conventional methodology by those skilled in the art. As appropriate, procedures involving the use of commercially available kits and reagents are generally carried out in accordance with manufacturer defined protocols and/or parameters unless otherwise noted.

The term "non-random" as used in the present invention, related to the pattern of acetylation of the chitosan obtained by the process of the invention, is used to define the pattern of acetylation obtained by the enzyme used in the process of the invention. The term "non-random" means that the pattern of acetylation of the chitosan prepared by the process of the invention is predictable and reproducible for determined process conditions and follows a non-Bernoulli distribution. The process conditions might slightly be modified but lead to the same or essentially the same chitosan pattern when the same enzyme is used. Also, slight modifications in the chitosan pattern may occur when reproducing a same process leading to essentially same chitosan, for example, leading to chitosan acetylation patterns having at least 90%, preferably at least 95%, more preferably at least 98% identity with each other. With other words, a non-random chitosan (with a determined pattern of acetylation) is obtained in a reproducible manner when a specific enzyme is used.

The term "polymer" as used in the present invention is used to define molecules having more than 30 monomer units. It differs from the term "oligomer", which, in the present invention, defines molecules having 30 monomer units or less, typically 25 or less. The term "chitosan oligomers", as used herein, thus refers to molecules that comprise no more than 30 monomeric units of D-glucosamine and *N*-acetyl-D-glucosamine. In various embodiments, such oligomers comprise 25 monomeric units or less, 20 units or less, 18 units or less, 16 units or less or 2 to 15 units. The lower limit is the dimer, i.e. a molecule that comprises two monomeric units. Such dimers are considered oligomers in the sense of the present invention. Similarly, the term "chitosan polymer", as used herein, thus refers to molecules that comprise more than 30 monomeric units of D-glucosamine and *N*-acetyl-D-glucosamine, typically more than 50, more than 100 or more than 150 units. The number of units can, in various embodiments, be as high as 5000, but is typically 3000 or less, 2000 or less, 1000 or less or in the range of 200-1000, 300-1000 or 500-1000.

The term "monomer" is used in the present invention with the meaning of a constitutional unit of a polymer and interchangeably with "subunit", "monomer residue", "residue", "repeat unit" of a polymer.

The term "structural unit" as used in the present invention means a sequence of specific monomers in the polymer.

The term "deacetylase" as used in the present invention in the term "chitin deacetylase enzyme" does not limit the function of the chitin enzyme as deacetylating. The term "chitin deacetylase enzyme" rather refers here to a common name given to the enzyme, discovered firstly as having deacetylating properties, for example in the nature. A chitin deacetylase enzyme may thus also show acetylating properties. Similarly, the term "chitosan acetylase" describes enzymes known for their acetylating activity on chitosan, but may also have deacetylation activity.

The term "chitosan polymer", as used in the present invention, defines a polymer of D-glucosamine units, wherein some D-glucosamine units may be acetylated whereas some others may be deacetylated. With other words, the term "chitosan polymer" will be used in the present invention without being limited to the fully deacetylated poly-D-glucosamine. In various embodiments, the degree of acetylation (DA) of the chitosan polymer is however not more than 30%, preferably 28% and less, 25% and less or 22% and less. It may also be advantageous that the chitosan polymer is not fully deacetylated so that the lower limit of the degree of acetylation may be 5% or more, 8% or more, 10% or more, or even 15%.

In contrast, the term "poly-D-glucosamine", as used herein, refers to the fully deacetylated chitosan that does not comprise any acetylated D-glucosamine units.

The term "chitin", as used herein, refers to a polymer comprising *N*-acetyl-D-glucosamine and D-glucosamine units, with the degree of acetylation being at least 50%, typically at least 70% or at least 80%. The term "poly-*N*-acetyl-*D*-glucosamine" is then used to designate a chitin that comprises 100% acetylated monomer units.

The term "GlcN" or "D" as used in the present invention stands for 2-amino-2-deoxy-β-D-glucose or D-glucosamine. If reference is made herein to "glucosamine", D-glucosamine is meant if not indicated otherwise.

The term "GlcNAc" or "A" as used in the present invention stands for 2-acetamido-2-deoxy-β-D-glucose or *N*-acetyl-D-glucosamine. If reference is made herein to "*N*-acetyl-glucosamine", *N*-Acetyl-*D-*glucosamine is meant if not indicated otherwise.

The term "degree of acetylation" or "DA", as used herein, is calculated as ΣA/(ΣA+ΣD)*100 to give the DA in percent (%), wherein in this formula ΣA is the number of GlcNAc units and ΣD the number of GlcN units in a given molecule. The degree of acetylation is, for example, determined by NMR, for example 1H-NMR or 13C-NMR, preferably 1H-NMR. Additionally or alternatively, the degree of acetylation can be determined by means of enzymatic hydrolysis of the chitosan polymer to monomers or small oligomers and quantitative mass spectrometry analysis. Methods to enzymatically hydrolyze a chitosan polymer are known in the art and can be chosen by those skilled in the art based on their general knowledge. In the case of chitosan oligomers, the preferred method for DA determination is mass spectrometry, but other methods such as, but not limited to NMR, Thin Layer Chromatography (TLC), or electrophoresis are also available. Still other methods include titration, dye binding assays, UV spectroscopy, and (FT)IR spectroscopy.

The term "degree of polymerization" or "DP", as used herein, refers to the number of monomeric units in a polymer or oligomer molecule. For polymers, it may also be given as the number average degree of polymerization which is given as DPₙ = Mₙ/M₀ with Mₙ being the number average molecular weight of the polymer and Mo being the molecular weight of a monomeric unit. The DP of chitosan polymers may be determined by Size Exclusion Chromatography (SEC), preferably SEC-RI-MALLS. The DP of chitosan oligomers may be determined by TLC, NMR, or, preferably, mass spectrometry.

The molecular weight of a polymer is, if not indicated otherwise, typically the number average molecular weight (Mₙ). This can be determined by a variety of methods known to those skilled in the art, such as, without limitation, GPC. In various embodiments, the molecular weight may also be determined using the method described in Niehues et al., "Chitosan analysis by enzymatic / mass spectrometric fingerprinting and in silico predictive modeling", Analytical Chemistry (2017) 89(22):12602-12608, which is incorporated herein by reference to the method described in C. Schatz, C. Viton, T. Delair, C. Pichot, and A. Domard, "Typical physicochemical behaviors of chitosan in aqueous solution", Biomacromolecules, (2003) 4: 641-648.

If amounts are given in % herein, these percentages refer to wt.-% if not indicated otherwise.

The term "essentially all" as used herein, means more than 60%, preferably more than 70%, more than 80% or more than 90% of the respective species in relation to the total amount of said type of compounds. Similarly, "essentially free of", as used herein, means that less than 10%, preferably less than 5%, less than 2% or less than 1% of the respective species in relation to the total amount of said type of compounds are comprised.

The invention is based on the inventors surprising finding that the hydrolysate of a chitosan polymer has different specific properties depending on the type of enzyme used for its production. More specifically, the inventors have found that oligomers produced by the action of a chitosanase have increased biostimulant activity and decreased antimicrobial activity. Furthermore, the inventors have found that a hydrolysate produced by action of a chitinase provides for improved antimicrobial activity but decreased biostimulant activity.

In a first aspect, the present invention thus relates to chitosan oligomers obtainable by enzymatic hydrolysis of a chitosan polymer by a chitosan hydrolase, wherein the chitosan oligomers have a degree of polymerization (DP) of 30 or less, preferably 20 or less.

The chitosan polymer used as a substrate for the enzymatic hydrolysis can be any chitosan polymer. Suitable chitosan polymers are commercially available, for example as a dietary supplement. While the chitosan used as a substrate may theoretically be produced by any method including the (partial) deacetylation of chitin or the partial acetylation of poly-D-glucosamine, the commercially most important method is the alkaline deacetylation of chitin, which is available in abundance from natural sources. Such processes commonly involve the use of sodium hydroxide, typically in form of an aqueous solution (40-50 wt.-%), and elevated temperatures and may be further supported by additional treatments, such as ultrasound treatment. Alternative methods include the enzymatic deacetylation of chitin and the enzymatic acetylation of poly-D-glucosamine.

For enzymatically deacetylating chitin or a chitosan polymer a chitin deacetylase may be used, for example a bacterial, fungal or viral chitin deacetylase, optionally a recombinant chitin deacetylase, preferably a fungal chitin deacetylase. Exemplary chitin deacetylases include, without limitation, those from *Colletotrichum lindemuthianum, Pestalotiopsis* sp., *Podospora anserina, Mucor rouxii, Puccinia graminis* f. sp. *tritici, Cryptococcus neoformans* or *Aspergillus niger.* In certain embodiments of the invention, a bacterial chitin deacetylase may be NodB from *Rhizobium* sp. GRH2 (NCBI acc. No. AJW76244.1), VcCDA from *Vibrio cholerae* (NCBI acc. No. AAF94439.1), or BcCDA5 from *Bacillus* sp., a viral chitin deacetylase may be (CvCDA) from the *Chlorovirus* CVK2, and a fungal chitin deacetylase may be CnCDA2 (EMBL Nucleotide Sequence Database acc. no. AJ938050) and CnCDA4 (fpd1/d25, Uniprot acc. no. Q96TR5) from *Crytococcus neoformans, ColLinCDA* from *Colletotrichum lindemuthianum* (Uniprot acc. no. AY633657), *An*CDA from *Aspergillus niger* (Uniprot acc. no. A2QZC8, AspGD ID An12g04480), MrCDA from *Mucor rouxii* (Uniprot acc. no. P50325), PesCDA from *Pestalotiopsis* sp. (NCBI acc. no. APH81274.1), *PgtCDA* from *Puccinia graminis* f. sp. *tritici* (NCBI acc. no. XP_003323413.1) or PaCDA from *Podospora anserina* (NCBI acc. no. CAP60162).

In various embodiments, it is preferred to use a chitosan polymer produced by alkaline deacetylation of chitin, as this produces a random pattern of acetylation in the chitosan polymer, which in turn gives rise to a population of diverse oligomers upon enzymatic hydrolysis.

In various embodiments, the chitosan polymer used as an educt is not fully deacetylated, but comprises a certain number of *N*-acetyl-D-glucosamine units. Typical are chitosan polymers with a degree of acetylation (DA) in % of at least 1, at least 2, at least 3, at least 4, at least 5, at least 7, at least 10 or at least 15 and up to 30 or up to 25. These are commonly obtained from the known methods of chitin deacetylation, as the reaction is typically incomplete.

The degree of polymerization (DP) of the chitosan polymers used is at least 30, typically 60 or more, 100 or more, or 150 or more. As defined above in relation to the term "polymer", the number of monomeric units is in principle not limited. In various embodiments, typical chitosan polymers have DP values of up to 5000, preferably up to 3000, up to 2000 or up to 1000. Specific ranges are from 100, 150, 200, 250 or 300 to 500, 600, 700, 800, 900 or 1000.

Alternatively or additionally, the average molecular weight Mw of the chitosan polymer may be comprised in the range from about 6.000 g/mol to about 1.000.000 g/mol, preferably from about 10.000 g/mol to 250.000 g/mol.

The oligomers of the invention are generated by enzymatic hydrolysis of the above-described chitosan polymers. The chitosan hydrolases used may be selected from any known chitosan hydrolase, typically from chitosanases and chitinases. The selection of the enzyme depends on the desired type of oligomer to be obtained. The chitosanases and chitinases may be selected from eukaryotic, for example mammalian, bacterial, viral and fungal enzymes. The enzymes may differ in their specificity and cleavage site.

Exemplary chitosanases suitable for use in accordance with the present invention include, without limitation, Csn-MN from *Bacillus spec MN,* Csn-MHKI from *Bacillus circulans MH-K1,* Csn-7M from *Bacillus spec. No.* 7-M, Csn-MN-VRE from *Bacillus spec.* MN, and CSN174 from *Streptomyces* N174. These are examples for three different types of chitosanases that differ in substrate specificity and cleavage site, as will be detailed below.

Exemplary chitinases suitable for use in accordance with the present invention include, without limitation, ChiB from *Serratia marcescens,* TvChi from *Trichoderma virens,* ChiG and ChiF from *Streptomyces coelicolor* A3(2), and Lysozyme and Chitotriosidase from *Homo sapiens.* These are examples for three different types of chitinases that differ in substrate specificity and cleavage site, as will be detailed below.

In various embodiments, the chitosan hydrolase used is a chitosanase. Typical motifs that are recognized and cleaved by suitable chitosanases include the following:
(1) [X]ₙDD I X[X]ₙ, or
(2) [X]ₙXD I D[X]ₙ, or
(3) [X]ₙDX I X[X]ₙ,
wherein X is Glucosamine (GlcN) or *N*-Acetyl-Glucosamine (GlcNAc), D is Glucosamine (GlcN), n is 0 or an integer of from 1 to 5000, preferably 1 to 1000, and I indicates the cleavage site/position.

Exemplary chitosanases that recognize motif (1) include, without limitation, Csn-MN from *Bacillus spec MN,* Csn-MHKI from *Bacillus circulans MH-K1,* and Csn-7M from *Bacillus spec. No. 7-M.* Exemplary chitosanases that recognize motif (2) include, without limitation, Csn-MN-VRE from *Bacillus spec.* MN. Exemplary chitosanases that recognize motif (3) include, without limitation, CSN174 from *Streptomyces* N174.

The chitosan oligomers obtained by using enzymes that recognize motif (1) therefore have the structure:
[X]ₙDD
wherein X is Glucosamine (GlcN) or *N*-Acetyl-Glucosamine (GlcNAc), D is Glucosamine (GlcN), A is N-Acetyl-Glucosamine (GlcNAc) and n is 0 or an integer of from 1 to 20, preferably 1 to 15, or 1 to 10. In various embodiments the DP of the oligomers is 16 or less, preferably 2-15, which requires n to be 14 or less or 0-13. In various embodiments, it is preferred that the oligomers comprise those with 3 or more monomeric units, i.e. the population of oligomers does not exclusively, preferably not predominantly or not essentially, consist of dimers. Furthermore, in various embodiments, such oligomers may comprise at least one A unit. It is apparent that the higher the degree of acetylation (DA) of the chitosan polymer used as the educt, the longer the oligomers become. The oligomers thus obtained have typically a degree of acetylation (DA) less than that of the chitosan polymer they are derived from, as the specificity of the enzyme leads to accumulation of the D units in the oligomers and of the A units in the remaining polymeric fraction if any. In various embodiments, the oligomers comprise those having the structure [X]ₙ₋₁ADD, wherein [X]ₙ₋₁ is free of DD motifs or is [A]ₙ₋₁. While some fraction of oligomers comprising DD motifs may remain, the more complete the enzymatic hydrolysis is, the lower the amount of oligomers with DD motifs becomes. In various embodiments, it may be that the total amount of oligomers produced, i.e. of molecules with a DP of 30 or less, comprises at least 30%, at least 50%, at least 60% or at least 70% by weight of oligomers of the formula [X]ₙ₋₁ADD, wherein [X]ₙ₋₁ is free of DD motifs or is [A]ₙ₋₁.

The same applies to chitosan oligomers obtained by using enzymes that recognize motif (2). These have the structure:
D[X]ₙXD, preferably D[X]ₙAD,
wherein X is Glucosamine (GlcN) or *N*-Acetyl-Glucosamine (GlcNAc), D is Glucosamine (GlcN), A is N-Acetyl-Glucosamine (GlcNAc) and n is 0 or an integer of from 1 to 20, preferably 1 to 10. In various embodiments the DP of the oligomers is 16 or less, preferably 2-15, which requires n to be 14 or less or 0-13. In various embodiments, it is preferred that the oligomers comprise those with 3 or more monomeric units, i.e. the population of oligomers does not exclusively, preferably not predominantly or not essentially, consist of dimers. Furthermore, in various embodiments, such oligomers may comprise at least one A unit. It is apparent that the higher the degree of acetylation (DA) of the chitosan polymer used as the educt, the longer the oligomers become. The oligomers thus obtained have typically a degree of acetylation (DA) less than that of the chitosan polymer they are derived from, as the specificity of the enzyme leads to accumulation of the D units in the oligomers and of the A units in the remaining polymeric fraction if any. In various embodiments, the oligomers comprise those having the structure the structure D[X]ₙAD, wherein [X]ₙ is free of DD motifs or is [A]ₙ. While some fraction of oligomers comprising DD motifs may remain, the more complete the enzymatic hydrolysis is, the lower the amount of oligomers with DD motifs becomes. In various embodiments, it may be that the total amount of oligomers produced, i.e. of molecules with a DP of 30 or less, comprises at least 30%, at least 50%, at least 60% or at least 70% by weight of oligomers of the formula D[X]ₙAD, wherein [X]ₙ is free of DD motifs or is [A]n.

The same applies to chitosan oligomers obtained by using enzymes that recognize motif (3). These have the structure:
[X]ₙDX,
wherein X is Glucosamine (GlcN) or *N*-Acetyl-Glucosamine (GlcNAc), D is Glucosamine (GlcN), A is N-Acetyl-Glucosamine (GlcNAc) and n is 0 or an integer of from 1 to 20, preferably 1 to 10. In various embodiments the DP of the oligomers is 16 or less, preferably 2-15, which requires n to be 14 or less or 0-13. In various embodiments, it is preferred that the oligomers comprise those with 3 or more monomeric units, i.e. the population of oligomers does not exclusively, preferably not predominantly or not essentially, consist of dimers. Furthermore, in various embodiments, such oligomers may comprise at least one A unit. It is apparent that the higher the degree of acetylation (DA) of the chitosan polymer used as the educt, the longer the oligomers become. The oligomers thus obtained have typically a degree of acetylation (DA) less than that of the chitosan polymer they are derived from, as the specificity of the enzyme leads to accumulation of the D units in the oligomers and of the A units in the remaining polymeric fraction if any. In various embodiments, the oligomers comprise those having the structure [X]ₙDD or [X]ₙDA, wherein [X]ₙ is [A]ₙ. While some fraction of oligomers comprising D units in the [X]ₙ part may remain, the more complete the enzymatic hydrolysis is, the lower the amount of oligomers with D units in said structural unit becomes. In various embodiments, it may be that the total amount of oligomers produced, i.e. of molecules with a DP of 30 or less, comprises at least 30%, at least 50%, at least 60% or at least 70% by weight of oligomers of the formula [A]ₙDX.

It has been surprisingly found that these oligomers and mixtures thereof demonstrate increased biostimulant activity and reduced antimicrobial activity. This is particularly pronounced if they are isolated from any remaining polymeric fraction. Such compositions and their respective use thus also form part of the invention and will be described in more detail below.

In various embodiments, the chitosan hydrolase used is a chitinase. Typical motifs that are recognized and cleaved by suitable chitinases include the following:
(1) [X]ₙXA I X[X]ₙ, or
(2) [X]ₙAX I A[X]ₙ, or
(3) [X]ₙAA I A[X]ₙ,
wherein X is Glucosamine (GlcN) or *N*-Acetyl-Glucosamine (GlcNAc), A is N-Acetyl-Glucosamine (GlcNAc), n is 0 or an integer of from 1 to 5000, preferably 1 to 1000, and I indicates the cleavage site/position.

Exemplary chitinases that recognize motif (1) include, without limitation, ChiB from *Serratia marcescens* and TvChi from *Trichoderma virens.* Exemplary chitinases that recognize motif (2) include, without limitation, ChiG and ChiF from *Streptomyces coelicolor* A3(2). Exemplary chitinases that recognize motif (3) include, without limitation, Lysozyme and Chitotriosidase from *Homo sapiens.*

The chitosan oligomers obtained by using chitinase enzymes that recognize motif (1) therefore have the structure:
[X]ₙXA
wherein X is Glucosamine (GlcN) or *N*-Acetyl-Glucosamine (GlcNAc), A is *N*-Acetyl-Glucosamine (GlcNAc) and n is 0 or an integer of from 1 to 20, preferably 1 to 15, or 1 to 10. In various embodiments the DP of the oligomers is 16 or less, preferably 2-15, which requires n to be 14 or less or 0-13. In various embodiments, it is preferred that the oligomers comprise those with 3 or more monomeric units, i.e. the population of oligomers does not exclusively, preferably not predominantly or not essentially, consist of dimers. Furthermore, in various embodiments, such oligomers may comprise at least one D unit. It is apparent that the higher the degree of acetylation (DA) of the chitosan polymer used as the educt, the shorter the oligomers become. The oligomers thus obtained have typically a degree of acetylation (DA) greater than that of the chitosan polymer they are derived from, as the specificity of the enzyme leads to accumulation of the A units in the oligomers and of the D units in the remaining polymeric fraction if any. In various embodiments, the oligomers comprise those having the structure [X]ₙDA, wherein [X]ₙ is [D]ₙ. While some fraction of oligomers comprising A units in the [X]ₙ unit may remain, the more complete the enzymatic hydrolysis is, the lower the amount of oligomers with such A units in the [X]ₙ part becomes. In various embodiments, it may be that the total amount of oligomers produced, i.e. of molecules with a DP of 30 or less, comprises at least 30%, at least 50%, at least 60% or at least 70% by weight of oligomers of the formula [D]ₙDA.

The same applies to chitosan oligomers obtained by using chitinase enzymes that recognize motif (2). These have the structure:
A[X]ₙAX,
wherein X is Glucosamine (GlcN) or *N*-Acetyl-Glucosamine (GlcNAc), A is *N*-Acetyl-Glucosamine (GlcNAc) and n is 0 or an integer of from 1 to 20, preferably 1 to 10. In various embodiments the DP of the oligomers is 16 or less, preferably 2-15, which requires n to be 14 or less or 0-13. In various embodiments, it is preferred that the oligomers comprise those with 3 or more monomeric units, i.e. the population of oligomers does not exclusively , preferably not predominantly or not essentially, consist of dimers. Furthermore, in various embodiments, such oligomers may comprise at least one D unit. It is apparent that the higher the degree of acetylation (DA) of the chitosan polymer used as the educt, the shorter the oligomers become. The oligomers thus obtained have typically a degree of acetylation (DA) greater than that of the chitosan polymer they are derived from, as the specificity of the enzyme leads to accumulation of the A units in the oligomers and of the D units in the remaining polymeric fraction if any. In various embodiments, the oligomers comprise those having the structure A[X]ₙAD, wherein A[X]ₙA is free of AXA motifs or [X]ₙ is [D]ₙ. While some fraction of oligomers comprising AXA motifs may remain, the more complete the enzymatic hydrolysis is, the lower the amount of oligomers with such AXA motifs becomes. In various embodiments, it may be that the total amount of oligomers produced, i.e. of molecules with a DP of 30 or less, comprises at least 30%, at least 50%, at least 60% or at least 70% by weight of oligomers of the formula A[X]ₙAD, wherein A[X]ₙA is free of AXA motifs or [X]ₙ is [D]ₙ.

The same applies to chitosan oligomers obtained by using chitinase enzymes that recognize motif (3). These have the structure:
A[X]ₙAA,
wherein X is Glucosamine (GlcN) or *N*-Acetyl-Glucosamine (GlcNAc), A is *N*-Acetyl-Glucosamine (GlcNAc) and n is 0 or an integer of from 1 to 20, preferably 1 to 10. In various embodiments the DP of the oligomers is 16 or less, preferably 2-15, which requires n to be 14 or less or 0-13. In various embodiments, it is preferred that the oligomers comprise those with 3 or more monomeric units, i.e. the population of oligomers does not exclusively, preferably not predominantly or not essentially, consist of dimers. Furthermore, in various embodiments, such oligomers may comprise at least one D unit. It is apparent that the higher the degree of acetylation (DA) of the chitosan polymer used as the educt, the shorter the oligomers become. The oligomers thus obtained have typically a degree of acetylation (DA) greater than that of the chitosan polymer they are derived from, as the specificity of the enzyme leads to accumulation of the A units in the oligomers and of the D units in the remaining polymeric fraction if any. In various embodiments, the oligomers comprise those having the structure A[X]ₙ₋₁DAA, wherein A[X]ₙ₋₁ is free of AAA motifs. While some fraction of oligomers comprising AAA motifs may remain, the more complete the enzymatic hydrolysis is, the lower the amount of oligomers with such AAA motifs becomes. In various embodiments, it may be that the total amount of oligomers produced, i.e. of molecules with a DP of 30 or less, comprises at least 30%, at least 50%, at least 60% or at least 70% by weight of oligomers of the formula A[X]ₙ₋₁ DAA, wherein A[X]ₙ₋₁ is free of AAA motifs.

It is generally preferred that the chitosan oligomers of the invention, irrespective of by which enzyme they have been produced, have a degree of polymerization (DP) of 16 or less, more preferably 2-15.

It is understood that enzymatic hydrolysis of chitosan polymers as described herein will yield a plurality of oligomers that differ in length and in structure/sequence. The invention explicitly encompasses these mixtures of oligomers that all satisfy the structural criteria defined herein, i.e. have an upper limit for DP and optionally satisfy the other structural criteria set forth herein. Accordingly, when reference is made herein to chitosan oligomers, said reference also includes the reference to chitosan oligomer mixtures and vice versa, with all embodiments described for the oligomers as such being similarly applicable to the mixtures of different oligomers. In various embodiments, such mixtures may comprise at least 2, 3, 4, 5, 6, 7, 8 or more different types of oligomers that differ in length and/or structure, i.e. sequence of monomeric A and D units. In various embodiments, such mixtures of oligomers comprise, in relation to the total amount of oligomers, at least 50%, preferably at least 70% or at least 80% or at least 90% by weight of the respective oligomers having the structures given above, i.e. (i) [X]ₙDD, D[X]ₙXD, preferably D[X]ₙAD, and/or [X]ₙDX; or (ii) [X]ₙXA, A[X]ₙAX, and/or A[X]ₙAA. It is of course also possible to generate mixtures of oligomers by using two or more different enzymes or combining the hydrolysates of two or more different enzymes.

In various embodiments, the mixture of chitosan oligomers comprises less than 50% by weight, relative to the total weight of oligomers and polymers of chitosan, of a chitosan polymer with a DP equal to or greater than 50. The amount may be even lower, i.e. less than 40%, less than 30%, less than 20%, less than 10% by weight or essentially free of such polymeric components. Said ranges may also apply to polymers with a DP of 30 or greater, or even to oligomers/polymers with a DP of 25 or greater or 20 or greater. Generally, in various embodiments, such mixtures of oligomers can be essentially free of polymers with a DP of 30 or more and optionally also free of monomers.

In various embodiments, the chitosan oligomers or mixtures thereof are obtained by subjecting the hydrolysate to a separation step to reduce the amount of residual polymer and/or the enzymes used and/or free monomers or other components of the reaction. Such steps may thus yield oligomeric chitosans or mixtures thereof in essentially pure form. Suitable separation techniques are known in the art and include, amongst other, chromatography techniques, such as FPLC, HPLC, semi-preparative HPLC, and preparative size exclusion chromatography (SEC), and dialysis.

As the enzymatic hydrolysis of the chitosan polymer leads to oligomer generation that differ in their degree of acetylation due to accumulation of D or A units, the difference in the degree of acetylation between the polymer used as the educt and the prepared oligomers is, in various embodiments, at least 1%, at least 2%, at least 3% or more.

In various embodiments, the concentration of the enzymes used is comprised in the range from 1 mU/mL to 20 mU/mL, preferably from 5 mU/mL to 10 mU/mL, preferably about 1 mU/ml (where 1 U = 1 mol reducing ends per minute). Alternatively defined, the concentration of the enzyme used is comprised in the range from 1 nM to 10 µM, preferably from 10 nM to 5 µM, preferably from 50 nM to 1.5 µM. The mass ratio enzyme to substrate (E/S) may be in the range from 10:1 to 1:100.000, wherein the substrate is the chitosan polymer.

In some embodiments, the concentration of the substrate is comprised in a range from 0.1 mg/ml to 10 mg/ml, preferably from 1 mg/ml to 3 mg/ml.

In some embodiments, the hydrolysis reaction is carried out in an aqueous solution. In certain embodiments, the hydrolysis reaction is carried out at a pH in the range from 4 to 10, preferably from 5 to 9. Alternatively or additionally, the hydrolysis reaction is performed at a temperature in the range from 25°C to 55°C, preferably about 40°C. Alternatively or additionally, the hydrolysis reaction is performed for a period of time in the range from 2 hours to 72 hours, for example from 5 hours to 48 hours, for example from 10 hours to 24 hours.

In certain embodiments, the reaction is carried out in the presence of other components. The other components may be one or more of buffers, one or more of organic solvents, or one or more of enzyme activators or co-factors.

In another aspect, the present invention relates to a composition comprising the chitosan oligomers or mixtures thereof described herein. Such a composition may be obtainable by isolating the oligomeric fraction of a chitosan polymer hydrolysate, for example by using separation steps as described above. In addition to the chitosan oligomers, the composition may further comprise additional components, such as at least one auxiliary or excipient. Also possible are diluents or solvents. The type and amount of such additional components may depend on the planned application or use and can be adapted accordingly. Formulation techniques and methods for different types of compositions envisaged herein, such as medicinal or agricultural compositions are well known to those skilled in the art and routinely practiced.

In still another aspect, the invention is directed to the use of the chitosan oligomers obtained by hydrolysis of a chitosan polymer with a chitosanase or mixtures thereof or compositions containing these oligomers or mixtures thereof, as a biostimulant, in particular for application to plants. The use may include increase of water or nutrient uptake or use efficiency, or increasing abiotic stress resistance to adverse conditions, such as drought and the like.

"Biostimulant", as used herein, relates to compounds as defined by the European Biostimulants Industry Council as: "contain[ing] substance(s) and/or micro-organisms whose function when applied to plants or the rhizosphere is to stimulate natural processes to enhance/benefit nutrient uptake, nutrient efficiency, tolerance to abiotic stress, and crop quality." Said effects are thus also meant and covered by the described use as biostimulant. Biostimulants act only a plant's vigor, offering no direct action against diseases, insects, or weeds.

Said aspect also relates to a method of stimulating natural processes to enhance/benefit nutrient uptake, nutrient efficiency, tolerance to abiotic stress, and crop quality, particularly in plants, by contacting them with (an effective amount of) the oligomers, oligomer mixtures or compositions of the invention. Said contacting may be by spraying or other known methods.

Alternatively or additionally, the use may include increasing the pathogen resistance of plants thus treated, for example to fungi, bacteria and viruses.

In a still further aspect, the invention relates to the use of chitosan oligomers obtained by hydrolysis of a chitosan polymer with a chitinase, mixtures thereof and compositions containing them as an antimicrobial agent or biopesticide. This use covers the growth inhibition of various pathogens, such as fungi, bacteria, viruses but also certain pests, such as insects, worms and the like.

Again, said aspect also relates to a method of inhibiting the growth of microbes and pests, particularly in relation to plant cultivation, by contacting them with (an effective amount of) the oligomers, oligomer mixtures or compositions of the invention. Said contacting may be by spraying or other known methods. Said aspect may also include post-harvest treatment.

The chitosan oligomers described herein, in particular those obtainable by hydrolysis of a chitosan polymer with a chitinase, may also be used as a medicament or may be used in the treatment of microbial infections. Said infections may be in a subject, for example a mammal, such as a human.

Said aspect also covers a method for treating a microbial infection in a subject, the method comprising administering an effective amount of the oligomers of the invention, mixture thereof or composition containing them.

Another use and method relate to use as a preservative, for example in cosmetic products, such as lotions, and food applications, including post-harvest treatment of plant products.

Various publications and/or references have been cited herein, the content of which are incorporated herein by reference.

### Examples

### Example 1:

The substrate specificities of the chitinolytic enzymes were determined by incubation of a chitosan polymer (DP around 1000, DA between 20 and 60%) until no further products were generated by the enzymes. These products were further *N*-acetylated by using [²H₆]acetic anhydride and on the one hand combined with [²H₃; ¹³C₄] acetylated glucosamine oligomers DP 1-6 (internal standard), and on the other hand dried, incubated with H₂¹⁸O at 70°C for 16h. The samples combined with the internal standard were further analyzed by LC-MS¹ to quantify the oligomers including their DA and DP, the ¹⁸O labeled samples were further analyzed by LC-MS² to quantitatively determine their pattern of acetylation. Afterwards, the results of the two analyses were combined to have quantitative data about the enzymatically produced COS including the DP, DA, and PA. This information allowed to decipher which sugar unit, GlcNAc or GlcN, was bound at the specific subsite in the active site of the enzyme during the hydrolysis.

**Table 1: Substrate specificities of the used chitosan hydrolases, the cleavage site is indicated by "I" and acetylation and deacetylation patterns of the products generated by enzymatic hydrolysis (left = non-reducing terminus, right = reducing terminus; A = GlcNAc, D = GlcN, X = GlcNAc or GlcN).**

| **Enzyme** | **Enzyme recognition site** | **Product structure** | **Enzyme example** |
|---|---|---|---|
| Chitinase 1 | [X]ₙ **X A I X** [X]ₙ | **X** [X]ₙ **X A** | ChiB (*Serratia macerescens*) *;* |
| | | | TvChi (*Trichoderma virens*) |
| Chitinase 2 | [X]ₙ **A X I A** [X]ₙ | **A** [X]ₙ **A X** | ChiG und ChiF (*Streptomyces coelicolor* A3(2)) |
| Chitinase 3 | [X]ₙ **A A I A** [X]ₙ | **A** [X]ₙ **A A** | Lysozym (*Homo sapiens*); |
| | | | Chitotriosidase (*Homo sapiens*) |
| Chitosanase 1 | [X]ₙ **D D** I **X** [X]ₙ | **X** [X]ₙ **D D** | Csn-MN (*Bacillus* sp. MN); |
| | | | Csn-MHKI (*Bacillus circulans* MH-K1); |
| | | | Csn-7M (*Bacillus* sp. No. 7-M) |
| Chitosanase 2 | [X]ₙ **X D I D** [X]ₙ | **D** [X]ₙ **X D** | Csn-MN-VRE *(Bacillus* sp. MN) |
| Chitosanase 3 | [X]ₙ **D X** I **X** [X]ₙ | **X** [X]ₙ **D X** | CSN174 (*Streptomyces* N174) |

**Table 2: Characteristics of the enzymatically produced hydrolysates of chitosan polymer (DA 20%) in comparison to hydrolysates obtained by acidic hydrolysis (left = non-reducing terminus, right = reducing terminus; A = GlcNAc, D = GlcN, X = GlcNAc or GlcN).**

| **Chitosan** | **Hydrolysate** | | **Polymer fraction** | | **Oligomer fraction** | | | **Ratio (Polymer / Oligomer)** |
|---|---|---|---|---|---|---|---|---|
| | **DP¹** | **DA [%]** | **DP** | **DA [%]** | **DP** | **DA [%]** | **PA** | |
| **Polymer DA 20%** | 210 | 20 | - | - | - | - | | - |
| **Acidic hydrolysate** | 25 | 19 | 100 | 20 | 2-15 | 20 | random | 78%/22% (3.5:1) |
| **Chitinase 1 Hydrolysat** | 5/60 | 21 | 150 | 11 | 2-15 | 37 | **X** [X]ₙ **X A** | 65% / 35% (2/1) |
| **Chitinase 2 Hydrolysat** | 5/30 | 20 | 150 | 14 | 2-15 | 32 | **A** [X]ₙ **A X** | 91%/9% (1/0,1) |
| **Chitosanase Hydrolysat** | 4/12 | 21 | 75 | 44 | 2-15 | 9 | **X** [X]ₙ **D D** | 28% / 72% (1/2) |

| | | | | | | | | |
|---|---|---|---|---|---|---|---|---|
| ¹ Maxima of the analysis via HP-SEC (See Figure 3) | | | | | | | | |

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. Chitosan oligomers obtainable by enzymatic hydrolysis of a chitosan polymer by a chitosan hydrolase, wherein the chitosan oligomers have a degree of polymerization (DP) of 30 or less, preferably 20 or less.

2. The chitosan oligomers of claim 1, wherein the chitosan polymer has a degree of acetylation (DA) of between 10 and 30, preferably 15 to 25, and a degree of polymerization (DP) of 60, 100 150 or more.

3. The chitosan oligomers of claim 1 or 2, wherein the chitosan hydrolase is a chitosanase.

4. The chitosan oligomers of claim 3, wherein the chitosanase specifically recognizes and cleaves the motif
(1) [X]ₙDD I X[X]ₙ, or
(2) [X]ₙXD I D[X]ₙ, or
(3) [X]ₙDX I X[X]ₙ,
wherein X is Glucosamine (GlcN) or *N*-Acetyl-Glucosamine (GlcNAc), D is Glucosamine (GlcN), n is 0 or an integer of from 1 to 5000, preferably 1 to 1000, and I indicates the cleavage position.

5. The chitosan oligomers of any one of claims 3 to 4, wherein the oligomers have the structure:
(1) [X]ₙDD, preferably [X]ₙ₋₁ADD, wherein [X]ₙ₋₁ is free of DD motifs or is [A]ₙ₋₁;
(2) D[X]ₙXD, preferably D[X]ₙAD, more preferably D[X]ₙAD, wherein [X]ₙ is free of DD motifs or is [A]ₙ; and/or
(3) [X]ₙDX, preferably [X]ₙDX, wherein [X]ₙ is [A]ₙ;
wherein X is Glucosamine (GlcN) or *N*-Acetyl-Glucosamine (GlcNAc), D is Glucosamine (GlcN), A is *N*-Acetyl-Glucosamine (GlcNAc) and n is 0 or an integer of from 1 to 20, preferably 1 to 10.

6. The chitosan oligomers of claim 1, wherein the chitosan hydrolase is a chitinase.

7. The chitosan oligomers of claim 6, wherein the chitinase specifically recognizes and cleaves the motif
(1) [X]ₙXA I X[X]ₙ,
(2) [X]ₙAX I A[X]ₙ, or
(3) [X]ₙAA I A[X]ₙ,
wherein X is Glucosamine (GlcN) or *N*-Acetyl-Glucosamine (GlcNAc), A is *N*-Acetyl-Glucosamine, n is 0 or an integer of from 1 to 5000, preferably 1 to 1000, and I indicates the cleavage position.

8. The chitosan oligomers of claim 7, wherein the oligomers have the structure:
(1) [X]ₙXA, preferably [X]ₙDA, wherein [X]ₙ is [D]ₙ;
(2) A[X]ₙAX, preferably A[X]ₙAD, wherein [X]ₙ is [D]ₙ; and/or
(3) A[X]ₙAA, preferably A[X]ₙ₋₁DAA, wherein A[X]ₙ₋₁ is free of AAA motifs;
wherein X is Glucosamine (GlcN) or *N*-Acetyl-Glucosamine (GlcNAc), D is Glucosamine (GlcN), A is *N*-Acetyl-Glucosamine (GlcNAc) and n is 0 or an integer of from 1 to 20, preferably 1 to 10.

9. The chitosan oligomers of any one of claims 1-8, wherein the oligomers have a degree of polymerization (DP) of 16 or less, more preferably 2-15.

10. The chitosan oligomers of any one of claims 1-9, wherein the chitosan oligomers are a mixture of chitosan oligomers that differ in length and/or structure, preferably comprising less than 50 % by weight relative to the total weight of chitosan oligomers and polymers of a chitosan polymer with a DP equal to or greater than 50.

11. Composition comprising the chitosan oligomers of any one of claims 1-10.

12. Use of a composition comprising the chitosan oligomers of any one of claims 3 to 5 or of a composition comprising said oligomers as a plant biostimulant.

13. Use of the composition comprising the chitosan oligomers of any one of claims 6-8 or of a composition comprising said oligomers as an antimicrobial agent and/or biopesticide.

14. Composition comprising the chitosan oligomers of any one of claims 6-8 or a composition comprising said oligomers for use as a medicament.

15. Composition comprising the chitosan oligomers of any one of claims 6-8 or a composition comprising said oligomers for use in the treatment of microbial infection.
